(19) Europäisches Patentamt
European Patent Office
Office européen des brevets

(11) Veröffentlichungsnummer: **0 030 331**
**B1**

(12) **EUROPÄISCHE PATENTSCHRIFT**

(45) Veröffentlichungstag der Patentschrift: **29.06.83**

(51) Int. Cl.³: **C 07 J 19/00,** A 61 K 31/705

(21) Anmeldenummer: **80107413.9**

(22) Anmeldetag: **27.11.80**

(54) **Neue C-3-verzweigte Cardenolid-glykoside, ihre Herstellung und Verwendung.**

(30) Priorität: **05.12.79 DE 2948835**

(43) Veröffentlichungstag der Anmeldung: **17.06.81 Patentblatt 81/24**

(45) Bekanntmachung des Hinweises auf die Patenterteilung: **29.06.83 Patentblatt 83/26**

(84) Benannte Vertragsstaaten: **AT BE CH DE FR GB IT LI NL**

(56) Entgegenhaltungen: **DE-A-2 517 293**

(73) Patentinhaber: **BASF Aktiengesellschaft, Carl-Bosch-Strasse 38, D-6700 Ludwigshafen (DE)**

(72) Erfinder: **Albrecht, Hans Peter, Dr., Brombeerweg 5, D-6940 Weinheim (DE)**

**EP 0 030 331 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Neue C-3-verzweigte Cardenolidglykoside, ihre Herstellung und Verwendung

Die vorliegende Erfindung betrifft neue C-3-verzweigte Cardenolidglykoside, ein Verfahren zu deren Herstellung sowie Arzneimittel, die diese Verbindungen enthalten, und deren Verwendung bei der Behandlung von Herzinsuffizienz.

Die bislang therapeutisch verwendeten Herzglykoside leiten sich von Steroiden der Cardenolid- oder der Bufadienolidreihe ab, die am C-3 eine β-ständige, sekundäre Hydroxylgruppe tragen. Über das Sauerstoffatom dieser Gruppe ist in den Glykosiden das steroidale Aglykon an einen Mono-, Di-, Tri- oder Tetrasaccharidrest gebunden. Trotz beträchtlicher Unterschiede im pharmakokinetischen Verhalten ist allen Verbindungen dieser Substanzklasse eine geringe therapeutische Breite gemeinsam, die ihre praktische Anwendung erschwert.

In der DE-OS Nr. 2517293 sind C-3-verzweigte Glykoside beschrieben, die sich vom Digitoxigenin ableiten und im Vergleich zu den bisher bekannten Glykosiden eine grössere therapeutische Breite aufweisen. Es wurden nun Substanzen gefunden, die ein verbessertes Wirkungsspektrum besitzen.

Die vorliegende Erfindung betrifft neue C-3-verzweigte Cardenolidglykoside der allgemeinen Formel I

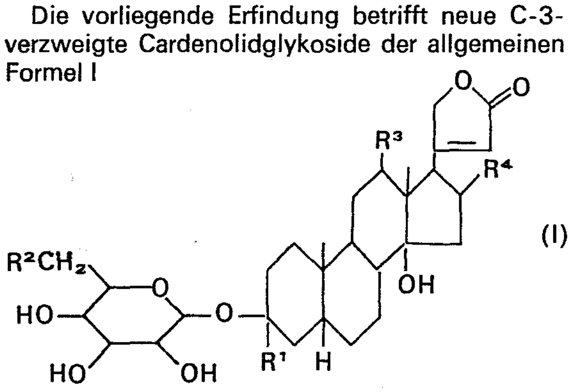

(I)

worin

$R^1$ ein Kohlenwasserstoffrest mit bis zu 4 Kohlenstoffatomen oder eine Phenylgruppe ist,

$R^2$ ein Wasserstoffatom oder eine Hydroxylgruppe bedeutet, und

$R^3$ und $R^4$ voneinander verschieden sind und ein Wasserstoffatom oder eine gegebenenfalls acylierte Hydroxylgruppe, wobei der Acylrest bis zu 7 Kohlenstoffatomen enthalten kann, darstellen.

Gegenstand der Erfindung ist ferner ein Verfahren zur Herstellung der C-3-verzweigten Cardenolidglykoside der Formel I, welches darin besteht, dass man eine Verbindung der Formel II

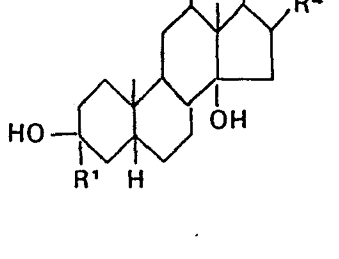

(II)

worin

$R^1$, $R^3$ und $R^4$ die oben angegebene Bedeutung besitzen, mit einer acylierten 1-Halogenhexopyranose der Formel III

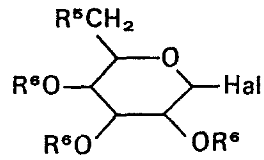

(III)

worin

$R^5$ ein Wasserstoffatom oder eine Acyloxygruppe mit 2 bis 8 Kohlenstoffatomen, und

$R^6$ eine Acylgruppe mit 2 bis 7 Kohlenstoffatomen bedeuten, in Gegenwart eines Säureakzeptors umsetzt und anschliessend die Acylgruppen am Hexopyranoserest und gegebenenfalls am C-12 oder C-16 abspaltet.

Die Umsetzung der steroidalen Aglykone der allgemeinen Formel II erfolgt zweckmässig mit einem Überschuss der acylierten 1-Halogenhexopyranose (3 bis 5 mol) bei Raumtemperatur in einem inerten Lösungsmittel, wie z.B. Methylenchlorid, 1,2-Dichloräthan, Äther, Tetrahydrofuran, Benzol, Acetonitril oder Nitromethan, vorzugsweise aber in 1,2-Dichloräthan oder Nitromethan.

Die Reaktionszeit beträgt in der Regel 12 bis 48 h. Als Säureakzeptor können Silber(I)oxid, Silber(I)carbonat, Quecksilber(II)oxid, Quecksilber(II)cyanid, gegebenenfalls auch Gemische aus Quecksilber(II)cyanid und Quecksilber(II)-bromid, verwendet werden. Bevorzugt wird Quecksilber(II)cyanid.

Zur Entacylierung eignen sich schwache Basen, wie z.B. methanolisches Ammoniak oder Kaliumhydrogencarbonat in wässerigem Methanol. Aufgrund der grossen Hydrolysebeständigkeit der sich am Steroidkern befindlichen O-Acylgruppen in 12- bzw. 16-Stellung im Vergleich zu den Acylgruppen des Zuckers, kann die Hydrolyse so gesteuert werden, dass selektiv die Monoacylverbindungen oder die vollständig entacylierten Produkte entstehen. Die Reaktionstemperatur liegt vorzugsweise bei 20 bis 70° C, die Reaktionsdauer bei 2 h bis 20 d.

Die Verbindungen der Formel II lassen sich analog dem in der DE-OS Nr. 2336445 angegebenen Verfahren aus den entsprechenden 3-Oxoverbindungen herstellen. Die Einführung einer Acylgruppe in der 12- oder 16-Stellung muss vor der Einführung des Hexopyranoserests erfolgen. Hierzu wird das Steroid mit einem Säurehalogenid oder Säureanhydrid in Gegenwart eines basischen Katalysators bei Raumtemperatur umgesetzt. Als Acylierungsmittel dient vorzugsweise Acetanhydrid/Pyridin.

Gegenstand der Erfindung sind weiter Arzneimittel enthaltend eine Verbindung der Formel I sowie die Verwendung der Verbindungen der Formel I bei der Behandlung der Herzinsuffizienz.

Die neuen Verbindungen steigern die Kontraktionskraft des Herzens und sind für die Therapie der Herzinsuffizienz geeignet.

Die therapeutische Breite der neuen Substanzen wurde an der Katze in Urethan/Chloralose-Narkose bestimmt. Während kontinuierlicher Glykosidzufuhr wurde aus der Veränderung des Herzinnendrucks gegen die Zeit dp/dt registriert. Die maximal inotrop wirksame Dosis (MID), Arrhythmiedosis (AD) und Letaldosis (LD) wurden zueinander ins Verhältnis gesetzt. Die dabei erhaltenen Werte liegen deutlich besser als die von Digoxin oder Gitoxin, die als Vergleichssubstanzen dienten.

Weiter wurde die Wirkdauer (Abklingquote) über die kumulativ toxische Wirkung bei i.v. Applikation an Katzen ermittelt.

Digoxin und Gitoxin weisen — in erster Linie bedingt durch den polaren Charakter der Aglykone im Vergleich zu Digitoxigenin — eine geringere enterale Wirksamkeit und Wirkdauer als die entsprechenden Digitoxigeninderivate auf. Überraschenderweise hat sich gezeigt, dass die erfindungsgemässen Verbindungen bei verbesserter therapeutischer Breite eine ausreichend lange Wirkdauer besitzen und oral wirksam sind. Die Verbindungen sind daher zur Therapie der chronischen Herzinsuffizienz hervorragend geeignet. Sie sollen vorzugsweise oral in den gängigen Applikationsformen wie Tabletten, Kapseln oder Dragées verabfolgt werden. Zur parenteralen Verabfolgung eignen sich sterile Lösungen. Der Dosierungsbereich liegt bei etwa 0,5 bis 5,0 mg pro Patient (80 kg) und Tag bei oraler Gabe.

Die nachstehenden Beispiele erläutern die Erfindung. Die Rf-Werte wurden an Kieselgelfertigplatten F 254 der Firma Merck, Darmstadt, mit Methylenchlorid/Aceton 5:1 (Laufmittel A), Methylenchlorid/Methanol 5:1 (Laufmittel B) oder Methylenchlorid/Methanol 10:1 (Laufmittel C) bestimmt.

*Beispiel 1:*

A. Herstellung des Ausgangsmaterials

a) Zu 5,6 g (14,4 mmol) 12β,14-Dihydroxy-3-oxo-5β,14β-card-20(22)-enolid in 350 ml abs. Tetrahydrofuran werden bei −60° C unter Rühren in einer Stickstoffatmosphäre langsam 80 ml (158 mmol) einer 1,6N Lösung von Methyllithium in Äther zugegeben. Man lässt das Reaktionsgemisch innerhalb von 2 h langsam auf 0° C kommen. Nach 2 h bei 0° C und weiteren 2 h bei Raumtemperatur wird die Reaktion durch Zugabe von 250 ml einer gesättigten wässerigen Ammoniumchloridlösung beendet. Es wird in 2000 ml Methylenchlorid aufgenommen, die organische Phase wird mit gesättigter wässeriger Natriumhydrogencarbonatlösung und Wasser gewaschen, über Natriumsulfat getrocknet und i. Vak. eingedampft.

Das Rohprodukt (6,8 g) wird durch Chromatographie an einer Kieselgelsäule mit Methylenchlorid/Methanol (20:1) aufgetrennt. In der Reihenfolge steigender Polarität werden zuerst 1,2 g Ausgangsmaterial isoliert. Weitere Elution gibt 2,8 g (65% d.Th.) 3β,12,14-Trihydroxy-3-methyl-5β,14β-card-20(22)-enolid:

Fp. = 220-240° C (Methylenchlorid/Äther)
$[\alpha]^{20}_{D}$ = +16,8° (c = 0,55, Chloroform)
Rf-Wert: 0,55 (Laufmittel B)

*Analyse:* $C_{24}H_{36}O_5$ (404,5)

| | | |
|---|---|---|
| berechnet: | C 71,25 | H 8,97% |
| gefunden: | C 70,9 | H 8,8% |

und 1,3 g (30% d.Th.) 3α,12β,14-Trihydroxy-3-methyl-5β,14β-card-20(22)-enolid:

Fp. = 250-265° C (Aceton/Äther)
$[\alpha]^{20}_{D}$ = +25,4° (c = 0,5, Chloroform/Äthanol 10:1)
Rf-Wert: 0,48 (Laufmittel B)

*Analyse:* $C_{24}H_{36}O_5$ (404,5)

| | | |
|---|---|---|
| berechnet: | C 71,25 | H 8,97% |
| gefunden: | C 71,3 | H 9,1% |

b) 1,5 g (3,7 mmol) 3β,12β,14-Trihydroxy-3-methyl-5β,14β-card-20(22)-enolid in 30 ml Pyridin werden mit 2,1 ml (22 mmol) Essigsäureanhydrid versetzt und 24 h bei Raumtemperatur stehengelassen. Das Reaktionsgemisch wurde unter Eiskühlung mit 2 ml Methanol versetzt und anschliessend i. Vak. eingedampft. Der Rückstand wurde in Methylenchlorid aufgenommen, und die Lösung nacheinander mit Kaliumhydrogensulfatlösung, Wasser, Natriumhydrogencarbonatlösung und Wasser gewaschen, über Natriumsulfat getrocknet und eingedampft. Kristallisation des Rückstandes aus Methylenchlorid/Äther/Hexan gab 1,3 g (79% d.Th.) 12β-Acetyl-3β,14-dihydroxy-3-methyl-5β,14β-card-20(22)-enolid:

Fp. = 180-184° C
$[\alpha]_D$ = +61,5° (c = 0,3, Chloroform)
Rf-Wert: 0,65 (Laufmittel B)

*Analyse:* $C_{26}H_{38}O_6$ (477)

| | | |
|---|---|---|
| berechnet: | C 69,93 | H 8,58% |
| gefunden: | C 69,8 | H 8,6% |

Entsprechend erhält man aus 1,05 g (2,6 mmol) 3α,12β,14-Trihydroxy-3-methyl-5β,14β-card-20(22)-enolid, 0,83 g (71% d.Th.) 12β-Acetyl-3α,14-dihydroxy-3-methyl-5β,14β-card-20(22)-enolid:

Fp. = 140-145° C (Methylenchlorid/Äther/Hexan)
$[\alpha]_D$ = +59° (c = 0,4, Chloroform)
Rf-Wert: 0,63 (Laufmittel B)

*Analyse:* $C_{26}H_{38}O_6$ (446,5)

| | | |
|---|---|---|
| berechnet: | C 69,93 | H 8,58% |
| gefunden: | C 69,7 | H 8,3% |

B. Herstellung des Endproduktes

a) 0,5 g (1,1 mmol) 12β-Acetyl-3β,14-dihydroxy-3-methyl-5β,14β-card-20(22)-enolid in 20 ml wasserfreiem 1,2-Dichloräthan werden mit 2,5 g Acetobromglucose, 2,0 g Quecksilber(II)cyanid und 0,05 g Quecksilber(II)bromid versetzt. Unter Ausschluss von Luftfeuchtigkeit und Durchleiten eines leichten Stickstoffstroms wird

16 h bei Raumtemperatur unter Rühren umgesetzt. Es wird filtriert, das Filtrat wird mit 800 ml Methylenchlorid versetzt und mit 20%iger wässeriger Kaliumiodidlösung (2 × 50 ml) und Wasser (2 × 50 ml) gewaschen, über Natriumsulfat getrocknet und eingedampft. Der Rückstand wird an einer Kieselgelsäule (Elution mit Methylenchlorid/Aceton 15:1) gereinigt. Man erhält 0,69 g (79% d.Th.) 12β-Acetyl-14-hydroxy-3-methyl-3β-[(2,3,4,6-tetra-O-acetyl-β-D-glycopyranosyl)oxy]-5β,14β-card-20(22)-enolid:

Fp. = 187-189° C
[α]$_D$ = +19,4° (c = 0,75, Chloroform)
Rf-Wert: 0,27 (Laufmittel A)

*Analyse:* $C_{40}H_{56}O_{15}$ (777)

| | | |
|---|---|---|
| berechnet: | C 61,84 | H 7,27% |
| gefunden: | C 61,6 | H 7,3% |

Entsprechend erhält man aus 0,76 g (1,7 mmol) 12β-Acetyl-3α,14-dihydroxy-3-methyl-5β,14β-card-20(22)-enolid 0,9 g (68% d.Th.) 12β-Acetyl-14-hydroxy-3-methyl-3α-[(2,3,4,6-tetra-O-acetyl-β-D-glucopyranosyl)oxy]-5β,14β-card-20(22)-enolid:

Fp. = 267-280° C (Methylenchlorid/Äther/Hexan)
[α]$_D$ = +38,3° (c = 0,5 in Chloroform)
Rf-Wert: 0,27 (Laufmittel A)

*Analyse:* $C_{40}H_{56}O_{15}$ (777)

| | | |
|---|---|---|
| berechnet: | C 61,84 | H 7,27% |
| gefunden: | C 61,8 | H 7,3% |

b) 1,0 g (1,3 mmol) 12β-Acetyl-14-hydroxy-3-methyl-3β-[(2,3,4,6-tetra-O-acetyl-β-D-glucopyranosyl)oxy]-5β,14β-card-20(22)-enolid werden mit 50 ml methanolischem Ammoniak versetzt. Nach 4 h bei Raumtemperatur wird i. Vak. eingedampft und der Rückstand an Kieselgel (Elution mit Methylenchlorid/Methanol 5:1) chromatographiert. Nach Kristallisation aus Methanol/Äther werden 0,53 g (68% d.Th.) 12β-Acetyl-14-hydroxy-3-methyl-3β-[(β-D-glucopyranosyl)-oxy]-5β,14β-card-20(22)-enolid erhalten:

Fp. = 178-180° C
[α]$_D$ = +31,5° (c = 0,35 in Methanol)
Rf-Wert: 0,13 (Laufmittel C)

*Analyse:* $C_{32}H_{48}O_{11}$ (609)

| | | |
|---|---|---|
| berechnet: | C 63,14 | H 7,95% |
| gefunden: | C 62,9 | H 8,2% |

Entsprechend erhält man 12β-Acetyl-14-hydroxy-3-methyl-3α-[(β-D-glucopyranosyl)oxy]-5β,14β-card-20(22)-enolid:

Fp. = 170-175° C (Methanol/Äther)
[α]$_D$ = +48° (c = 0,35 in Methanol)
Rf-Wert: 0,12 (Laufmittel C)

*Analyse:* $C_{32}H_{48}O_{11}$ (609)

| | | |
|---|---|---|
| berechnet: | C 63,14 | H 7,95% |
| gefunden: | C 62,8 | H 8,4% |

*Beispiel 2:*

Analog Beispiel 1 erhält man 16β-Acetyl-14-hydroxy-3-methyl-3β-[(β-D-glucopyranosyl)-oxy]-5β,14β-card-20(22)-enolid:

Fp. = 162-167° C (Methanol/Äther)
Rf-Wert: 0,16 (Laufmittel C)

*Analyse:* $C_{32}H_{48}O_{11}$ (609)

| | | |
|---|---|---|
| berechnet: | C 63,14 | H 7,95% |
| gefunden: | C 63,0 | H 7,9% |

16β-Acetyl-14-hydroxy-3-methyl-3α-[(β-D-glucopyranosyl)oxy]-5β,14β-card-20(22)-enolid:

Fp. = 168-170° C (Methanol/Äther)
Rf-Wert: 0,17 (Laufmittel C)

*Analyse:* $C_{32}H_{48}O_{11}$ (609)

| | | |
|---|---|---|
| berechnet: | C 63,14 | H 7,95% |
| gefunden: | C 62,8 | H 7,8% |

*Beispiel 3:*

a) Zu 0,5 g (1,1 mmol) 12β-Acetyl-3β,14-dihydroxy-3-methyl-5β,14β-card-20(22)-enolid in 20 ml 1,2-Dichloräthan werden 2,5 g Tri-O-benzoyl-α-L-rhamnopyranosylbromid (vgl. Beispiel 1A, b), 2,0 g Quecksilber(II)cyanid und etwa 3 g Molekularsieb A 4 zugegeben. Unter Ausschluss von Luftfeuchtigkeit und Durchleiten eines leichten Stickstoffstroms wird 16 h bei Raumtemperatur unter Rühren umgesetzt. Nach Aufarbeitung wie in Beispiel 1 angegeben, werden 0,87 g (86% d.Th.) 12β-Acetyl-14-hydroxy-3-methyl-3β-[(2,3,4-tri-O-benzoyl-α-L-rhamnopyranosyl)oxy]-5β,14β-card-20(22)-enolid als Schaum erhalten:

Rf-Wert: 0,48 (Laufmittel A)

*Analyse:* $C_{53}H_{60}O_{13}$ (905)

| | | |
|---|---|---|
| berechnet: | C 70,33 | H 6,68% |
| gefunden: | C 70,1 | H 6,7% |

b) 0,87 g (0,96 mmol) 12β-Acetyl-14-hydroxy-3-methyl-3β-[(2,3,4-tri-O-benzoyl-α-L-rhamnopyranosyl)oxy]-5β,14β-card-20(22)-enolid in 20 ml Methanol werden mit 4 ml 20%iger wässeriger Kaliumhydrogencarbonatlösung versetzt. Nach 4 d bei Raumtemperatur wird mit verdünnter Salzsäure neutralisiert und i. Vak. eingedampft. Chromatographie an einer Kieselgelsäule (Elution mit Methylenchlorid/Methanol 15:1) gab 0,11 g (19% d.Th.) 12β-Acetyl-14-hydroxy-3-methyl-3β-[(α-L-rhamnopyronosyl)oxy]-5β,14β-card-20(22)-enolid:

Fp. = 214-218° C (Methanol/Äther)
[α]$_D$ = + 13° (c = 0,3 in Methanol)
Rf-Wert: 0,38 (Laufmittel B)

*Analyse:* $C_{32}H_{48}O_{10}$ (593)

| | | |
|---|---|---|
| berechnet: | C 64,84 | H 8,16% |
| gefunden: | C 64,5 | H 8,3% |

Anschliessend werden mit Methylenchlorid/Methanol 5:1 3,0 g (56% d.Th.) 12β,14-Dihydroxy-3-methyl-3β-[(α-L-rhamnopyranosyl)oxy]-5β,14β-card-20(22)-enolid erhalten:

Fp. = 255-260° C (Methanol/Äther)
[α]$_D$ = −8° (c = 0,4 in Methanol)
Rf-Wert: 0,25 (Laufmittel B)

*Analyse:* $C_{30}H_{46}O_9$ (551)
berechnet: C 65,43 H 8,42%
gefunden: C 65,1 H 8,1%

*Beispiel 4:*

1,0 g (1,3 mmol) 12β-Acetyl-14-hydroxy-3-methyl-3β-[(2,3,4,6-tetra-O-acetyl-β-D-glucopyranosyl)oxy]-5β,14β-card-20(22)-enolid (vgl. Beispiel 1B, a) in 20 ml Methanol werden bei Raumtemperatur mit 4 ml 20%iger wässeriger Kaliumhydrogencarbonatlösung versetzt. Der Verlauf der Entacetylierung wird mittels DC (Laufmittel C) verfolgt. Nach 6 d werden nochmals 3 ml 20%ige wässerige Kaliumhydrogencarbonatlösung zugegeben. Nach insgesamt 10 d Reaktionsdauer wird mit verdünnter Salzsäure neutralisiert, i. Vak. eingedampft und der Rückstand an einer Kieselgelsäule (Elution mit Methylenchlorid/Methanol 5:1) chromatographiert. Man erhält 0,35 g (48% d.Th.) 12β,14-Dihydroxy-3-methyl-3β-[(β-D-glucopyranosyl)oxy]-5β,14β-card-20(22)-enolid:
Fp. = 231-233° C (Methanol/Äther)
$[\alpha]_D$ = +12,5° (c = 0,4 in Methanol)
Rf-Wert: 0,17 (Laufmittel B)

*Analyse:* $C_{30}H_{46}O_{10}$ (567)
berechnet: C 63,58 H 8,18%
gefunden: C 63,2 H 8,2%

Analog erhält man 12β,14-Dihydroxy-3-methyl-3α-[(β-D-glucopyranosyl)oxy]-5β,14β-card-20(22)-enolid:
Fp. = 174-179° C (Methanol/Äther)
$[\alpha]_D$ = +14° (c = 0,14 in Methanol)
Rf-Wert: 0,17 (Laufmittel B)

*Analyse:* $C_{30}H_{46}O_{10}$ (567)
berechnet: C 63,58 H 8,18%
gefunden: C 63,10 H 8,1%

*Beispiel 5:*

A. Herstellung des Ausgangsmaterials

a) Ausgehend von 4,5 g (11,6 mmol) 14,16β-Dihydroxy-3-oxo-5β,14β-card-20(22)-enolid werden durch Umsetzung mit Methyllithium und säulenchromatographische Trennung der beiden Isomeren analog wie in Beispiel 1 beschrieben zunächst 2,7 g (57% d.Th.) 3β,14,16β-Trihydroxy-3-methyl-5β,14β-card-20(22)-enolid erhalten:
Fp. = 219-230° C (Methylenchlorid/Äther/Hexan)
$[\alpha]^{20}_D$ = +40,4° (c = 0,65 in Chloroform/Methanol 1:1)
Rf-Wert: 0,31 (Laufmittel C)

*Analyse:* $C_{24}H_{36}O_5$ (404,5)
berechnet: C 71,25 H 8,97%
gefunden: C 71,2 H 9,0%

Weitere Elution mit Methylenchlorid/Methanol 15:1 gibt 1,1 g (23% d.Th.) 3α,14,16β-Trihydroxy-3-methyl-5β,14β-card-20(22)-enolid:
Fp. = 208-220° C (Methylenchlorid/Äther/-Hexan)
$[\alpha]^{20}_D$ = +42,7° (c = 0,5 in Chloroform/Methanol 1:1)
Rf-Wert: 0,20 (Laufmittel C)

*Analyse:* $C_{24}H_{36}O_5$ (404,5)
berechnet: C 71,25 H 8,97%
gefunden: C 71,1 H 8,8%

Analog Beispiel 1A, b, werden aus 1,4 g (3,5 mmol) 3β,14,16β-Trihydroxy-3-methyl-5β,14β-card-20(22)-enolid 1,2 g (79% d.Th.) 16β-Acetyl-3β,14-dihydroxy-3-methyl-5β,14β-card-20(22)-enolid erhalten:
Fp. = 127-130° C (Methylenchlorid/Äther)
$[\alpha]_D$ = −3° (c = 0,6 in Chloroform)
Rf-Wert: 0,45 (Laufmittel C)

*Analyse:* $C_{26}H_{38}O_6$ (446,5)
berechnet: C 69,93 H 8,58%
gefunden: C 69,5 H 8,3%

Entsprechend erhält man aus 0,7 g (1,7 mmol) 3α,14,16β-Trihydroxy-3-methyl-5β,14β-card-20(22)-enolid 0,55 g (72% d.Th.) 16β-Acetyl-3α,14-dihydroxy-3-methyl-5β,14β-card-20(22)-enolid:
Fp. = 230-238° C (Methylenchlorid/Äther/-Hexan)
$[\alpha]_D$ = −12,8° (c = 0,55 in Chloroform)
Rf-Wert: 0,55 (Laufmittel C)

*Analyse:* $C_{26}H_{38}O_6$ (446,5)
berechnet: C 69,93 H 8,58%
gefunden: C 69,5 H 8,3%

B. Herstellung des Endproduktes

a) Wie in Beispiel 1B, a, werden aus 0,42 g (0,95 mmol) 16β-Acetyl-3α,14-dihydroxy-3-methyl-5β,14β-card-20(22)-enolid 0,49 g (67% d.Th.) 16β-Acetyl-14-hydroxy-3-methyl-3α-[(2,3,4,6-tetra-O-acetyl-β-D-glucopyranosyl)oxy]-5β,14β-card-20(22)-enolid erhalten:
Fp. = 125-129° C (Methylenchlorid/Äther/-Hexan)
$[\alpha]_D$ = −9° (c = 0,5 in Chloroform)
Rf-Wert: 0,34 (Laufmittel A)

*Analyse:* $C_{40}H_{56}O_{15}$ (777)
berechnet: C 61,84 H 7,27%
gefunden: C 61,4 H 7,4%

Ebenso erhält man aus 1,1 g (2,46 mmol) 16β-Acetyl-3β,14-dihydroxy-3-methyl-5β,14β-card-20(22)-enolid 1,2 g (63% d.Th.) 16β-Acetyl-14-hydroxy-3-methyl-3β-[(2,3,4,6-tetra-O-acetyl-β-D-glucopyranosyl)oxy]-5β,14β-card-20(22)-enolid:
Fp. = 221-230° C (Methylenchlorid/Äther/-Hexan)
$[\alpha]_D$ = −6° (c = 0,5 in Chloroform)
Rf-Wert: 0,42 (Laufmittel A)

*Analyse:* $C_{40}H_{56}O_{15}$ (777)
berechnet: C 61,84 H 7,27%
gefunden: C 61,6 H 7,3%

b) Analog Beispiel 4 erhält man aus den unter a aufgeführten Verbindungen:

16β,14-Dihydroxy-3-methyl-3β-[(β-D-glucopyranosyl)oxy]-5β,14β-card-20(22)-enolid:
Fp. = 240-246° C (Methanol/Äther)
$[\alpha]_D$ = +18° (c = 0,4 in Methanol)
Rf-Wert: 0,17 (Laufmittel B)

*Analyse:* $C_{30}H_{46}O_{10}$ (567)

| | | |
|---|---|---|
| berechnet: | C 63,58 | H 8,18% |
| gefunden: | C 63,3 | H 8,2% |

16β,14-Dihydroxy-3-metyhl-3α-[(β-D-glucopyranosyl)oxy]-5β,14β-card-20(22)-enolid:
Fp. = 166-168° C (Methanol/Äther)
$[\alpha]_D$ = +17° (c = 0,4 in Methanol)
Rf-Wert: 0,16 (Laufmittel B)

*Analyse:* $C_{30}H_{46}O_{10}$ (567)

| | | |
|---|---|---|
| berechnet: | C 63,58 | H 8,18% |
| gefunden: | C 63,4 | H 8,3% |

**Patentansprüche** für die Vertragsstaaten: BE, CH, DE, FR, GB, IT, LI, NL

1. C-3-verzweigte Cardenolidglykoside der Formel (I)

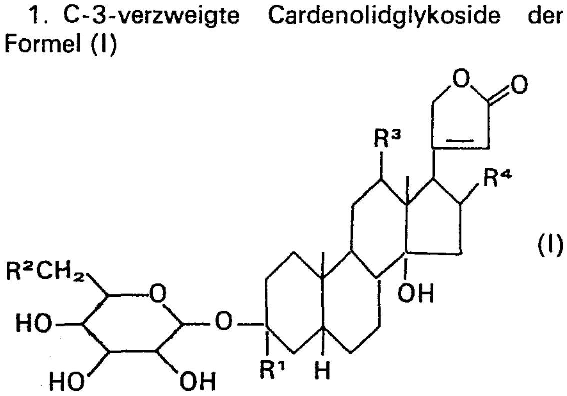

(I)

worin
$R^1$ ein Kohlenwasserstoffrest mit bis zu 4 Kohlenstoffatomen oder eine Phenylgruppe ist,
$R^2$ ein Wasserstoffatom oder eine Hydroxylgruppe bedeutet, und
$R^3$ und $R^4$ voneinander verschieden sind und ein Wasserstoffatom oder eine gegebenenfalls acylierte Hydroxylgruppe, wobei der Acylrest bis zu 7 Kohlenstoffatomen enthalten kann, darstellen.

2. Verfahren zur Herstellung der C-3-verzweigten Cardenolidglykoside der Formel (I), gemäss Anspruch 1, dadurch gekennzeichnet, dass man eine Verbindung der Formel (II)

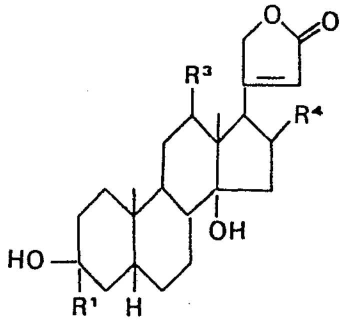

(II)

worin $R^1$, $R^3$ und $R^4$ die oben angegebene Bedeutung besitzen, mit einer acylierten 1-Halogenhexopyranose der Formel (III)

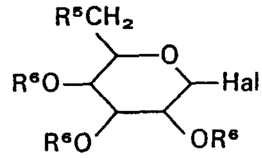

(III)

worin
$R^5$ ein Wasserstoffatom oder eine Acyloxygruppe mit 2 bis 8 Kohlenstoffatomen, und
$R^6$ eine Acyloxygruppe mit 2 bis 7 Kohlenstoffatomen bedeuten, in Gegenwart eines Säureakzeptors umsetzt, und anschliessend die Acylgruppe am Hexopyranoserest und gegebenenfalls am C-12 oder C-16 abspaltet.

3. Arzneimittel enthaltend eine Verbindung gemäss Anspruch 1.

4. Die Verbindungen gemäss Anspruch 1 zur Anwendung bei der Bekämpfung von Herzinsuffizienz.

**Patentanspruch** für den Vertragstaat: AT

Verfahren zur Herstellung C-3-verzweigter Cardenolidglykoside der Formel (I)

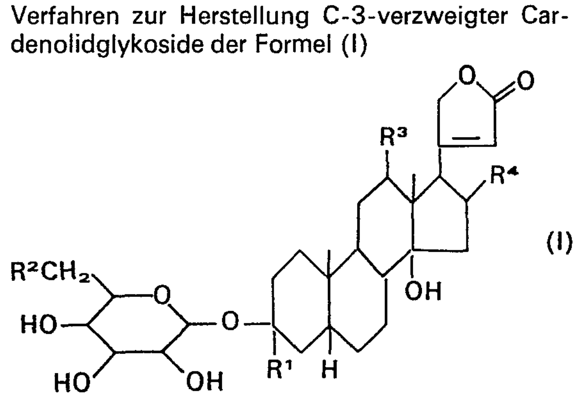

(I)

worin
$R^1$ ein Kohlenwasserstoffrest mit bis zu 4 Kohlenstoffatomen oder eine Phenylgruppe ist,
$R^2$ ein Wasserstoffatom oder eine Hydroxylgruppe bedeutet, und
$R^3$ und $R^4$ voneinander verschieden sind und ein Wasserstoffatom oder eine gegebenenfalls acylierte Hydroxylgruppe, wobei der Acylrest bis zu 7 Kohlenstoffatomen enthalten kann, darstellen, dadurch gekennzeichnet, dass man eine Verbindung der Formel (II)

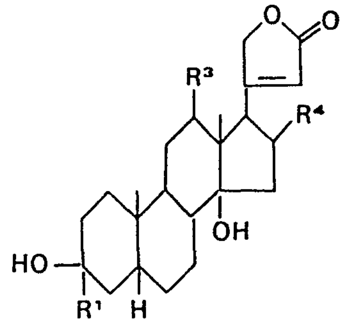

(II)

worin $R^1$, $R^3$ und $R^4$ die oben angegebene Bedeutung besitzen, mit einer acylierten 1-Halogenhexopyranose der Formel (III)

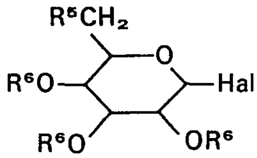

(III)

worin

$R^5$ ein Wasserstoffatom oder eine Acyloxygruppe mit 2 bis 8 Kohlenstoffatomen, und

$R^6$ eine Acyloxygruppe mit 2 bis 7 Kohlenstoffatomen

bedeuten, in Gegenwart eines Säureakzeptors umsetzt, und anschliessend die Acylgruppen am Hexopyranoserest und gegebenenfalls am C-12 oder C-16 abspaltet.

**Claims** for the contracting States: BE, CH, LI, DE, FR, GB, IT, NL

1. A $C_3$-branched cardenolideglycoside of the formula (I)

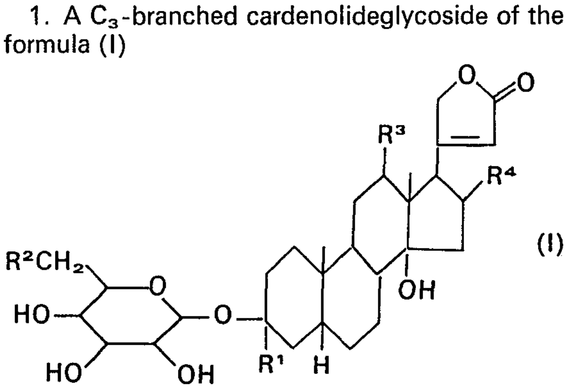

(I)

where

$R^1$ is a hydrocarbon radical of up to 4 carbon atoms, or phenyl,

$R^2$ is hydrogen or hydroxyl, and

$R^3$ and $R^4$ are different from one another and denote hydrogen or an optionally acylated hydroxyl, it being possible for the acyl to contain up to 7 carbon atoms.

2. A process for the preparation of a $C_3$-branched cardenolideglycoside of the formula (I) as claimed in claim 1, wherein a compound of the formula (II)

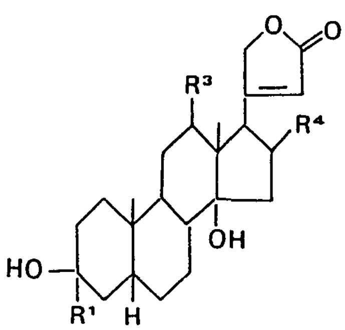

(II)

where $R^1$, $R^3$ and $R^4$ have the above meanings, in reacted in the presence of an acid acceptor with an acylated 1-halohexopyranose of the formula (III)

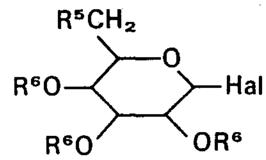

(III)

where $R^5$ is hydrogen or acyloxy of 2 to 8 carbon atoms, and $R^6$ is acyloxy of 2 to 7 carbon atoms, and then the acyl groups which are present on the hexopyranose radical and may be present on the 12th or 16th carbon atom are eliminated.

3. A drug containing a compound as claimed in claim 1.

4. A compound as claimed in claim 1 for use in the treatment of cardiac insufficiency.

**Claim** for the contracting State: AT

A process for the preparation of a $C_3$-branched cardenolideglycoside of the formula (I)

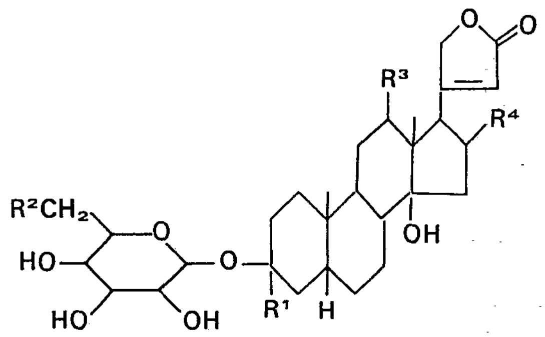

(I)

where

$R^1$ is a hydrocarbon radical of up to 4 carbon atoms, or phenyl,

$R^2$ is hydrogen or hydroxyl, and

$R^3$ and $R^4$ are different from one another and denote hydrogen or an optionally acylated hydroxyl, it being possible for the acyl to contain up to 7 carbon atoms,

wherein a compound of the formula (II)

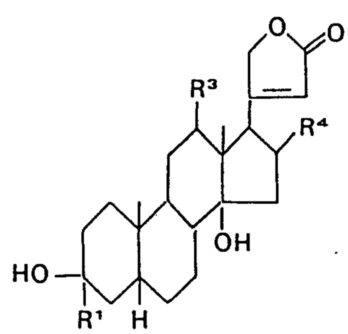

(II)

where $R^1$, $R^3$ and $R^4$ have the above meanings, is reacted in the presence of an acid acceptor with an acylated 1-halohexopyranose of the formula (III)

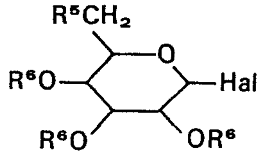

(III)

where $R^5$ is hydrogen or acyloxy of 2 to 8 carbon atoms, and $R^6$ is acyloxy of 2 to 7 carbon atoms, and then the acyl groups which are present on the hexopyranose radical and may be present on the 12th or 16th carbon atom are eliminated.

**Revendications** pour les Etats contractants BE, CH, LI, DE, FR, GB, IT, NL

1. Cardénolideglucosides ramifiés sur l'atome de carbone 3, de la formule (I)

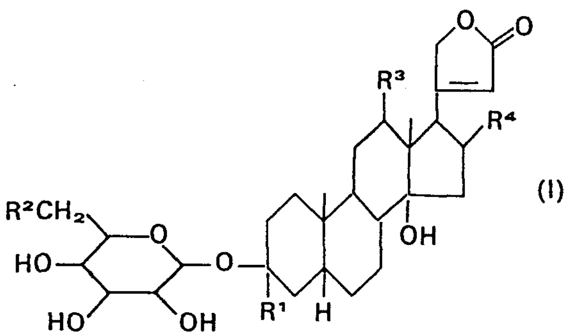

(I)

dans laquelle

$R^1$ désigne un groupe hydrocarboné avec jusqu'à 4 atomes de carbone ou un groupe phényle,

$R^2$ désigne un atome d'hydrogène ou un groupe oxhydryle, et

$R^3$ et $R^4$, qui diffèrent l'un de l'autre, représentent chacun un atome d'hydrogène ou un groupe oxhydryle éventuellement acylé par un groupe acyle pouvant comporter jusqu'à 7 atomes de carbone.

2. Procédé de préparation des cardénolideglucosides de la formule (I) suivant la revendication 1, caractérisé en ce que l'on fait réagir un composé de la formule (II)

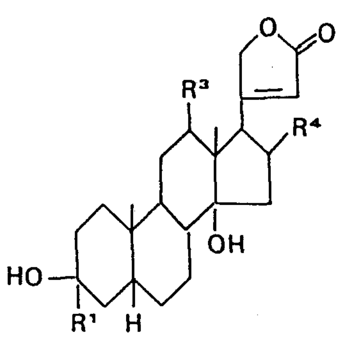

(II)

dans laquelle $R^1$, $R^3$ et $R^4$ possèdent la signification définie plus haut, en présence d'un fixateur d'acide avec un halo-1 hexopyrannose acylé de la formule (III)

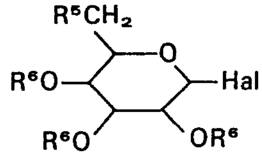

(III)

dans laquelle

$R^5$ désigne un atome d'hydrogène ou un groupe acyloxy en $C_2$ à $C_8$, et

$R^6$ désigne un groupe acyloxy en $C_2$ à $C_7$, puis on élimine les groupes acyle sur la fraction hexopyrannose et éventuellement sur l'atome de carbone 12 ou sur l'atome de carbone 16.

3. Médicament, contenant un composé suivant la revendication 1.

4. Utilisation des composés suivant la revendication 1 pour le traitement de l'insuffisance cardiaque.

**Revendication** pour l'Etat contractant: AT

Procédé de préparation de cardénolideglucosides ramifiés sur l'atome de carbone 3, de la formule (I)

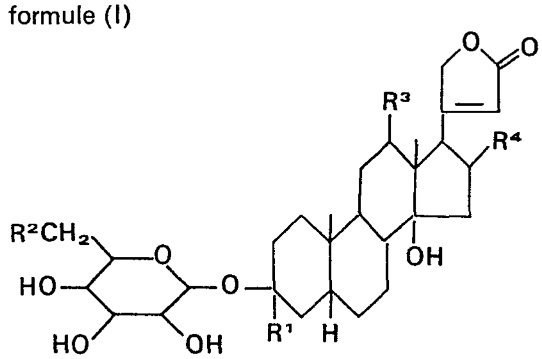

(I)

dans laquelle

$R^1$ désigne un groupe hydrocarboné avec jusqu'à 4 atomes de carbone ou un groupe phényle,

$R^2$ désigne un atome d'hydrogène ou un groupe oxhydryle, et

$R^3$ et $R^4$, qui diffèrent l'un de l'autre, représentent chacun un atome d'hydrogène ou un groupe oxhydryle éventuellement acylé par un groupe acyle pouvant comporter jusqu'à 7 atomes de carbone,

caractérisé en ce que l'on fait réagir un composé de la formule (II)

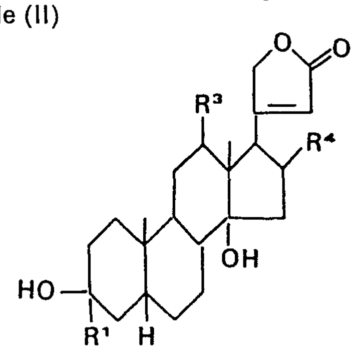

(II)

dans laquelle $R^1$, $R^3$ et $R^4$ possèdent la signification définie plus haut, en présence d'un fixateur d'acide avec un halo-1 hexopyrannose acylé de la formule (III)

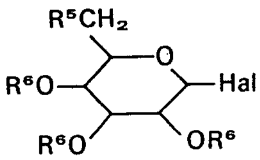

(III)

dans laquelle

$R^5$ désigne un atome d'hydrogène ou un groupe acyloxy en $C_2$ à $C_8$, et

$R^6$ désigne un groupe acyloxy en $C_2$ à $C_7$, puis on élimine les groupes acyle sur la fraction hexopyrannose et éventuellement sur l'atome de carbone 12 ou sur l'atome de carbone 16.